# EUROPEAN PATENT SPECIFICATION

(11) **EP 1 776 080 B1**
(45) Date of publication and mention of the grant of the patent: **14.01.2015**
(21) Application number: 05760190.8
(22) Date of filing: 15.06.2005
(51) Int. Cl.: A61K 6/10, A61C 9/00

(54) **METHOD OF PREPARING DENTITION FOR DENTAL IMPRESSION**
VERFAHREN ZUR HERSTELLUNG VON GEBISSEN FÜR ABFORMUNGEN
PROCEDE DE PREPARATION DE LA DENTITION POUR UNE PRISE D'IMPRESSION DENTAIRE

(30) Priority: 16.06.2004 US 580053 P
(43) Date of publication of application: 25.04.2007
(73) Proprietor: DENTSPLY International Inc., York, PA 17405-0872 (US)
(72) Inventor: BOGHOSIAN, Alan, Ara, Glenview, IL 60025-4138 (US)
(74) Representative: Hartz, Nikolai
(86) International application number: PCT/US2005/021157
(87) International publication number: WO 2006/007370

(56) References cited:
- EP-A- 1 188 419
- US-A- 4 395 398
- US-A- 4 617 950
- US-A- 5 554 028
- MILLAR B J, DUNNE S M, ROBINSON P B: "The effect of a surface wetting agent on void formation in impressions" JOURNAL OF PROSTHETIC DENTISTRY, vol. 77, 1997, pages 54-56, XP008053394
- MILLAR B J, DUNNE S M, ROBINSON P B: "An in vivo study of a clinical surfactant used with poly(vinyl siloxane) impression materials" QUINTESSENCE INTERNATIONAL, vol. 27, no. 10, 1996, pages 707-709, XP008053393

## Description

### Technical Field

The present invention generally relates to the taking of dental impressions. More particularly, the invention relates to the conditioning of dentition substantially immediately prior to the taking of the dental impression to wet-out the dentition. More specifically the invention relates to the application of a wetting agent, a surface treating agent or other impression preparatory agent to the dentition prior to the taking of a dental impression when using a polyvinyl, silicone or other rubber or elastomeric impression material. The inventive material may also include hemostatic materials or functional silicone polymers that can co-polymerize with the tray (impression) material.

### Background of the Invention

It is known to apply a wetting agent to a tooth preparation prior to the taking of an impression with hydrocolloid impression materials such as polyethers. In recent years the use of hydrocolloids has decreased in favor of polyvinyl, silicone or other rubber or elastomeric impression materials. A drawback to the use of these dental impression materials has been their hydrophobicity, making it difficult to take precise impressions of the details of the tooth and/or hard tissue when it is wetted with blood, saliva, or other fluids. When making the impression, the blood, saliva, or other fluids are forced into the margins of the teeth or pits and fissures in the teeth by the hydrophobic silicone impression material, rendering it difficult to take detailed and precise impressions because of the high surface tension of the materials. The dental practitioner may attempt to dry the oral cavity by blowing air into the oral cavity, but this is cumbersome not only for the practitioner but also the patient, particularly where the patient is bleeding. The hydrophobicity of dental impression materials also prevents the formation of accurate models formed from gypsum slurries.

Repeated attempts have been made to render silicone dental impression materials more hydrophilic by including various ionic or non-ionic surfactants in the composition, as is described for example in DE 4129613 to Hefner et al., U S Pat No. 4,657,959 to Bryan et al, U. S. Pat. No. 4,691,039 to Aasen et al., and U.S. Pat. No. 4,752,633 to Aasen et al U. S. Pat. No. 4,778,832 to Futami discloses use of a protein additive such as albumin as well as a silicone oil or non-ionic surfactant to increase hydrophicity While these materials have met with some success, the additives sometimes suffer from certain drawbacks, including instability in moist air, deactivation of platinum catalyst complexes, and reduction in the tear strength of the dental impression For example, they may swell first in water, and then dissolve gradually, and undergo phase separation, such as in vinyl-terminated polydimethylsiloxane base components.

U. S. Pat. No. 5,534,560 describes an attempt to modify the impression material rather than wetting the dentition As such, this patent describes a variation of the prior art that involves changing the chemistry of the impression material to make it less hydrophobic.

Millar B-J., Dunnes S. M., Robinson P.B.; QUINTESSENCE INTERNATIONAL, vol. 27, no. 10, 1996, 707-709 disclose an in vivo study of a clinical surfactant used with poly(vinyl siloxane) impression materials.

A need exists therefore, for a method of preparing or conditioning dentition to receive or physically contact a dental impression material in order to affect a detailed and accurate impression. That is, the need exists for a material that will wet the dentition as opposed to modifying the impression material. The method should be useful with both hydrophobic and hydrophilic dental impression materials. The method should also be useful in assisting the practitioner in removing the impression material from the dentition.

### Summary of the Invention

The present invention provides a method according to claim 1.

In general, a method of conditioning dentition before the taking of a dental impression includes the step of applying a wetting agent to the dentition. The wetting agent is a surfactant mixture with a dentally suitable carrier. The wetting agent may also include a hemostatic or vasoconstrictor agent or functional silicone polymers that can co-polymerize with the tray (impression) material .All such materials shall be collectively referred to herein as "wetting agent" or "wetting agents" or the like unless otherwise specified.

A method of taking a dental impression includes the step of applying such a wetting agent to the dentition before contacting the dentition with a polyvinyl, silicone or other rubber or elastomeric impression material The impression material may be hydrophobic or hydrophilic

### Preferred Embodiments for Carrying Out the Invention

The present invention provides a method of taking a dental impression that includes conditioning the dentition of which it is desired to make an impression. The inventive method includes applying a wetting agent to the dentition and then applying the impression material. The invention has usefulness with any dental impression material, but is particularly suitable for use with polyvinyl and silicone or other rubber or elastomeric impression materials. The invention has an additional particular usefulness with hydrophilic dental impression materials but is suitable for use with hydrophobic materials as well.

The wetting agent is preferably a surfactant or a surfactant-like material, all such materials referred to as "surfactants" unless otherwise noted. By "surfactant" and "surfactant-like" it is meant any material that serves to reduce liquid-to-liquid surface tension. The surfactant should be suitable for use in the oral cavity, such as Igepal co-530, a nonyl phenoxy-poly (ethyleneoxy) ethanol. Other suitable surfactants include for example Alkyl Amine Ethoxylates; Alkyl Polyglucosides; Branched Secondary Alcohol Ethoxylates; Ethylene Oxide/Propylene Oxide Copolymers; Low Foam Surfactants; Nonylphenol Ethoxylates (NPE); Octylphenol Ethoxylates; Secondary Alcohol Ethoxylates and branched Secondary Alcohol Ethoxylates and Alkoxylates; Alkoxylates; Alkyldiphenyloxide Disulfonate Salts; Dioctyl Sulfosuccinates; Phosphate Esters; Sulfates, Polyether Sulfates, and Sulfonates; Phosphate Esters; Alkyldiphenyloxide Disulfonic acids and salts; chelating surfactants; n-Acyl-sarcosines/n-Acyl Sarcosinates; Ethylene Oxide/Propylene Oxide Copolymers; polyacrylates; anionic and nonionic surfactants; polyoxyethylene polyoxypropylene block copolymers; PLURONIC (BASF); sodium lauryl sulfate; TWEEN 20, which is a trademark of ICl America; nonionic surfactants such as a water soluble polyoxyethylene monoester of sorbitol with a C.sub.10-18 fatty acid ester of sorbitol (and sorbitol anhydrides), consisting predominantly of the monoester, condensed with about 10-30, preferably about 20 moles of ethyleneoxide, the fatty acid (aliphatic hydrocarbon-monocarboxylic acid) may be saturated or unsaturated, e.g. lauric, palmitic, stearic, oleic acids. TWEEN 20 (which is a polyoxyethylene (20) sorbitan monolaurate); Anionic surfactants are also useful such as water soluble salts of higher fatty acid monoglyceride monosulfates, as sodium salts of the monosulfated monoglycerides, or hydrogenated coconut oil fatty acids, higher alkylsulfates, such as sodium lauryl sulfate and alkyl aryl sulfonates, such as sodium dodecyl benzene sulfonate; and the like. Other surfactants such as fluorinated surfactants and surface tension reducing materials may also be incorporated within the compositions. Still other useful surfactants include egg albumin, sarcosinate surfactants, isethionate surfactants and taurate surfactants; and those described for example in US Pat. Nos. Suitable optional surfactants are described more fully in U.S. Pat. No. 3,959,458, May 25, 1976 to Agricola et al.; U.S. Pat. No. 3,937,807, Feb. 10, 1976 to Haefele; and U.S. Pat. No. 4,051,234, Sep. 27, 1988 to Gieske et al..

It is preferred to include an orally suitable carrier with the wetting agent, such as a water soluble or partially water soluble substance such as water, glycerin, glycerol or a polyol or the like.

The wetting agent material may also include other components such as an additive including one or more of hemostatic, vasoconstrictor, anesthetic, desensitizing and flavoring agents. The material may also include thickening agents, diluents or fillers to modify the viscosity of the material as may be desired. Chemical initiators, accelerators, retardants or setting modifiers may also be added to the wetting agent to control the rate of setting reaction at the interface of the impression material and the dentition. It is also useful to include a colorant in the wetting agent to aid in determining its location on the dentition. Solvents, evaporating agents or spreading agents may also be added to promote a uniform distribution of the wetting agent on the dentition.

In delivering the wetting agent, any suitable device or method maybe employed, such as by using a brush, an aerosol foam composition or an aerosol spray or atomized mist preparation. It may also be useful to introduce the wetting agent into a pressurized stream of fluid or gas to direct the wetting agent to the dentition. The material may be supplied in bulk a bottle or in a unit dose configuration. A setting or gelling agent may be employed.

It has been found that using a wetting agent as in the present invention, a lower film thickness may be achieved in the impression material. This allows the impression material to flow into sub-gingival parts of the preparation. Historically, capturing good sub-gingival detail is in part limited by the lack of flow of the impression material. The application of the pre-impression wetting agent according to the present invention would create a very hydrophilic surface that would enable a very hydrophilic impression material (such as Aquasil Ultra available from DENTSPLY International) or even somewhat hydrophilic material to flow easier into these difficult to reach sub-gingival places. Also if a dental clinician was able achieve better impression material flow through the application of a surface treatment agent, excessive or substantially reduced gingival retraction is achievable. This substantially eliminates or reduces patient tissue trauma that can occur during the retraction process.

As an example, a wetting agent having 3 weight percent of Igepal CO 520 was mixed with about 3 weight percent of aluminum chloride (Hemogin-L from VanR) in an aqueous solution. The solution may be buffered or unbufferred. When applied to dentition prior to taking an impression, the dentition was wetted to the extent that an improved flow of the impression material onto dentition surfaces was observed. It is preferred to employ from about 0 to about 25 weight percent of a homeostatic agent such as aluminum chloride, aluminum sulfate or the like. Similarly, although 100 weight by weight of a surfactant may be employed in the present invention, a 3 weight percent in water is one preferred composition. It is also understood that other components such as the homeostatic agent may also provide a surface tension reducing effect, and hence may be employed in the present invention either with or as a substitute to traditional surfactants. Hence, any material that will reduce the surface tension by wetting the dentition is within the scope of the invention.

The viscosity of the present invention may be changed as desired, as long as a film thickness is achieved that is conducive to the impression to be made.

As stated herein, the wetting agent may also include a material that "sets" or "cures" or itself polymerize with the impression material with which it is used. In practice, the inventive material is applied to the dentition and then the impression material is placed onto the dentition in the normal manner. The wetting agent will then polymerize or set (cross branching) with the impression material. This setting or polymerizing of the wetting agent may be accomplished by any means including by chemical and light energy interactions. The wetting material may be formulated such that it is induced to polymerize or set by exposure to the same chemical or light curing of the impression material. A wetting agent according to the invention may include a cure package, a photosensitive cure package or the like. The wetting agent may also physically mix with the impression material such that upon curing it becomes an intimate and physically mixed part thereof.

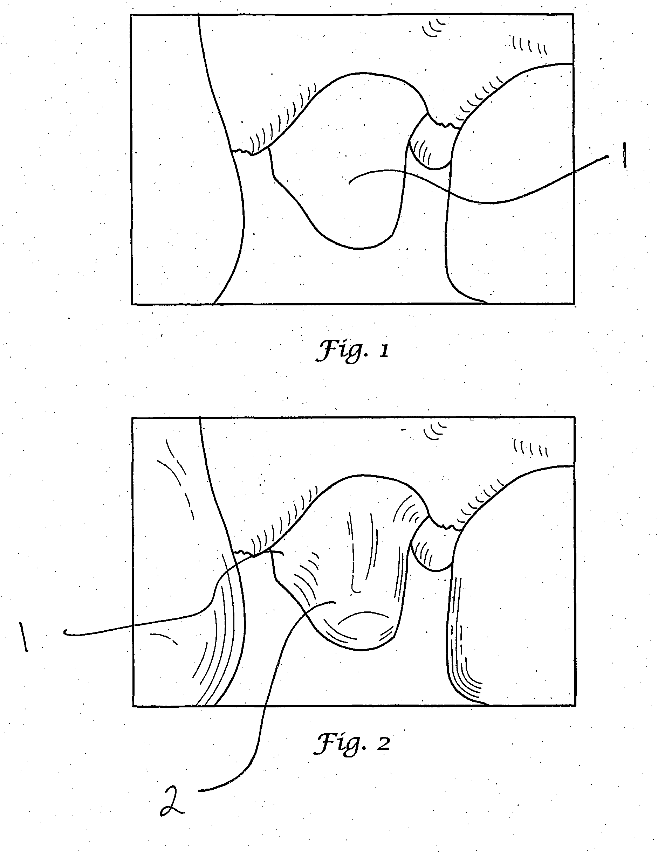

The wetting agent as described above was employed substantially immediately prior to the taking of a dental impression as follows. Following tooth (1 in Fig. 1) preparation and prior to taking the impression, the agent (2 in Fig. 2) is "painted" or otherwise applied onto the entire preparation in an isolated environment (usually with cotton rolls as is standard practice). Before the application of the impression material (such as but not necessarily a low viscosity material) the isolated preparation would be blown with a stream of air, thus leaving a very thin film of the surfactant based pre-impression surface agent on the tooth. The impression material applied to this surface will now flow freely on the surface. When the wetting agent also contains a hemostatic agent, commonly used gingival retraction cord can be soaked with the agent prior to placement using a standard single or double cord technique. If bleeding would occur during the placement of the cords, the packed preparations would be rinsed free of all blood and saliva, isolated properly and then the agent would be re-applied to the entire surface of the preparation as well as the placed cords. The impression process would the follow by the removal of the cord, application of a stream of air, and then syringing of the impression material on the preparation with the thin film.

When gingival retraction cord is removed from a dried tooth and sulcus, the material used to manufacture the cord, such as cotton, often sticks to the tissue (as does a cotton roll placed in the cheek). For tissues that are not healthy or are fragile, this often can initiate bleeding. With the present invention, because the impression is taken in a controlled wet environment, this sticking does not occur or is at least substantially reduced with cord removal. Additionally, the use of glycerin or other similar agents further acts as a lubricant when placing as well a removing the cord. The wetting agent may also be used with the retraction cord as either in the form being soaked prior to placement or it may be supplied to the user in a pretreated manner. Application of the wetting agent to the cord can also provide a decreased surface tension to the gingival areas thus allowing enhanced flow of the impression material around the margins.

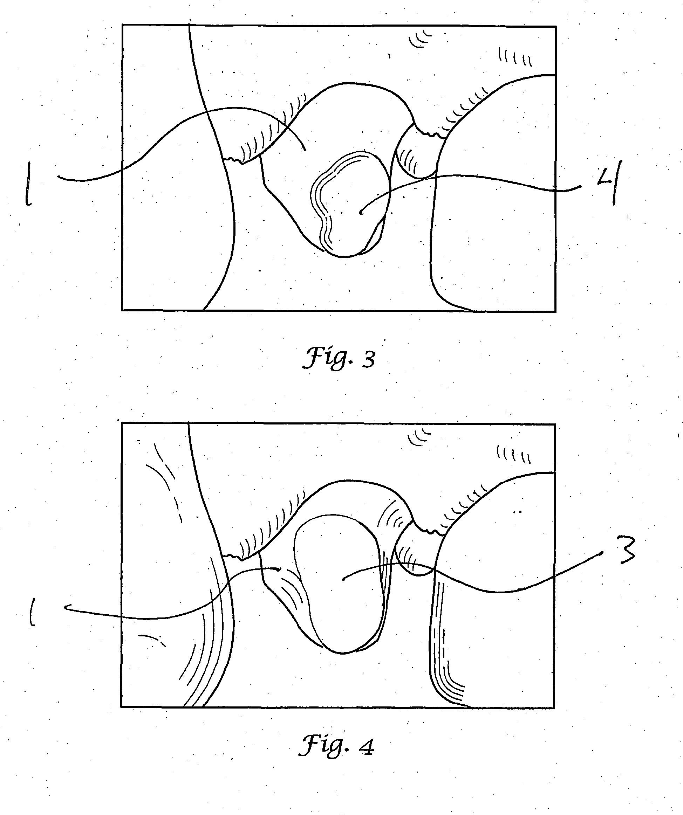

As a comparison, a dental impression was made using the same impression material but without the step of first conditioning the dentition with the wetting agent. When compared, the impression 3 made according to the present invention (Fig. 4) showed improved impression material flow than the material 4 applied without the inventive wetting agent (Fig. 3). If a hydrophilic material is placed on dry surface, the benefit of the hydrophilic impression material is limited. However, if a controlled hydrophilic film is applied to the tooth and preferably employing the same surfactant as may be present in the impression material itself, "like" surfaces would permit greater flow.

The wetting material according to the invention may also include additives such as flavorants, scenting agents, viscosity modifiers, preservatives, antioxidants, coloring agents and the like.

While the invention has been exemplified herein with respect to impressions of natural dentition, it will be appreciated that the invention has equal applicability to impressions of other devices found in the oral cavity. For example, according to the invention improved impressions can be made of conventional dental appliances such as orthodontic appliances, implants, restorations and the like. Often these items are fabricated from metals and/or polymeric, synthetic or other naturally occurring materials. It is to be further appreciated that improved impressions obtainable according to the invention are desirable in the dental arts and that the invention otherwise provides an advancement thereof.

While the invention has been exemplified herein with respect to impressions of natural dentition, it will be appreciated that the invention has equal applicability in providing void free stone models when casting impressions. As an example, voids are common occurrences when pouring models; especially from impressions taken with elastomers that lack sufficient hydrophilicity. This is especially seen when casting impressions with long narrow tooth preparations. Reduction of the surface tension will permit enhanced flow of stone and other casting materials.

It is apparent therefore, that the inventive use of a wetting agent when taking a dental impression improves the adaptation of the impression material to the dentition. The invention has been described herein without attempting to describe all of the embodiments thereof as may be within the scope of the invention and included variations will be apparent to those skilled in the art. The scope of the invention shall be determined only by any attached claims.

## Claims

1. A method of taking a dental impression of a dentition including sub-gingival parts, comprising the steps of:
(i) conditioning the dentition including sub-gingival parts by the application thereto of a wetting agent comprising a surfactant and a carrier;
(ii) contacting the dentition with a dental impression material selected from the group consisting of hydrophilic and hydrophobic dental impression materials, whereby the impression material is allowed to flow into sub-gingival parts,
further comprising the step of preparing the dentition with a gingival retraction cord, wherein said gingival retraction cord has been contacted with a wetting agent further comprising a hemostatic agent.

2. A method as in claim 1, wherein said surfactant is a nonyl phenoxy-poly (ethyleneoxy)ethanol.

3. A method as in claim 1, wherein said dental impression material is a polyvinyl material.

4. A method as in claim 1 wherein the impression material is an elastomer.

5. A method as in claim 1 wherein said wetting agent includes an additive selected from the group consisting of hemostatic, vasoconstrictor, anesthetic, desensitizing and flavoring agents.

## Patentansprüche

1. Verfahren zum Herstellung eines Dentalabdrucks einer Dentition, einschließlich subgingivaler Bereiche, umfassend die Schritte:
(i) Konditionierung der Dentition, einschließlich subgingivale Bereiche, durch Anwendung eines Netzmittels, umfassend ein Tensid und einen Träger;
(ii) Kontaktieren der Dentition mit einem Dentalabdruckmaterial, ausgewählt aus der Gruppe, bestehend aus hydrophilen und hydrophoben Dentalabdruckmaterialien, wobei das Abdruckmaterial in subgingivale Bereiche einfließen gelassen wird,
ferner umfassend einen Schritt einer Präparation der Dentition mit einem Zahnfleischretraktionsfaden, wobei der Zahnfleischretraktionsfaden mit einem Netzmittel kontaktiert wurde, das ein hämostatisches Mittel enthält.

2. Das Verfahren nach Anspruch 1, wobei das Tensid ein Nonylphenoxy-poly(ethylenoxy)ethanol ist.

3. Das Verfahren nach Anspruch 1, wobei das dentale Abdruckmaterial ein Polyvinylmaterial ist.

4. Das Verfahren nach Anspruch 1, wobei das Abdruckmaterial ein Elastomer ist.

5. Verfahren nach Anspruch 1, wobei das Netzmittel ein Additiv, ausgewählt aus der Gruppe, bestehend aus hämostatischen, vasokonstriktiven, anästhetischen, desensibilisierenden und aromatisierenden Mitteln, beinhaltet.

## Revendications

1. Procédé pour prendre une empreinte dentaire d'une dentition comprenant des parties sous-gingivales, comprenant les étapes consistant à :
(i) conditionner la dentition comprenant les parties sous-gingivales en y appliquant un agent de mouillage comprenant un agent tensio-actif et un agent porteur ;
(ii) mettre en contact la dentition avec une matière d'empreinte dentaire choisie dans le groupe comprenant les matières d'empreinte dentaire hydrophiles et hydrophobes, moyennant quoi la matière d'empreinte est autorisée à s'écouler dans les parties sous-gingivales,
comprenant en outre l'étape consistant à préparer la dentition avec un fil de rétraction gingivale, ledit fil de rétraction gingivale ayant été en contact avec un agent de mouillage comprenant en outre un agent hémostatique.

2. Procédé selon la revendication 1, dans lequel ledit agent tensio-actif est un nonylphénoxypoly(éthylène-oxy)éthanol.

3. Procédé selon la revendication 1, dans lequel ladite matière d'empreinte dentaire est une matière polyvinylique.

4. Procédé selon la revendication 1, dans lequel la matière d'empreinte est un élastomère.

5. Procédé selon la revendication 1, dans lequel ledit agent de mouillage comprend un additif choisi dans le groupe comprenant les agents hémostatiques, vasoconstricteurs, anesthésiants, désensibilisants et aromatisants.
